# EUROPEAN PATENT SPECIFICATION

(11) **EP 1 624 058 B1**
(45) Date of publication and mention of the grant of the patent: **25.05.2011**
(21) Application number: 04008224.0
(22) Date of filing: 05.04.2004
(51) Int. Cl.: C12N 15/10, C12P 19/34, C12Q 1/68

(54) **Methods and compositions for performing template dependent nucleic acid primer extension reactions that produce a reduced complexity product**
Verfahren und Zusammensetzungen zur Durchführung Matrizen-abhängiger Nukleinsäure-Primer-Verlängerungsreaktionen, wodurch ein Produkt mit reduzierter Komplexität hergestellt wird
Procédés et compositions pour réaliser des réactions d'extension d'amorces nucléiques à partir d'une matrice et dont le produit présente une complexité réduite

(30) Priority: 03.06.2003 US 454686
(43) Date of publication of application: 08.02.2006
(73) Proprietor: Agilent Technologies, Inc., Santa Clara CA 95051 (US)
(72) Inventor: Ilsley-Tyree, Diane, San Jose, California 95123 (US); Amorese, Douglas A., Los Altos, California 94022 (US)
(74) Representative: Barth, Daniel Mathias

(56) References cited:
- US-A- 5 554 516
- US-A- 5 716 785
- US-A- 5 830 662
- US-A- 5 888 819
- US-A- 6 132 997
- OMORI Y ET AL: "Comparative PCR: A Simple and Sensitive Method for Quantifying Low-Abundance mRNA Species" GENOMICS, ACADEMIC PRESS, SAN DIEGO, US, vol. 67, no. 2, 15 July 2000 (2000-07-15), pages 140-145, XP004437885 ISSN: 0888-7543

## Description

### INTRODUCTION

### Technical Field

The technical field of this invention is differential gene expression analysis.

### Background of the Invention

The characterization of cellular gene expression finds application in a variety of disciplines, such as in the analysis of differential expression between different tissue types, different stages of cellular growth or between normal and diseased states. The goal of many gene expression profiling applications is to detect low abundant messages within a given population and to measure the relative distribution of all messages within a given population while using the smallest possible sample amount of input nucleic acid. Achievement of this goal is complicated by the vast differences in the distribution of mRNA in a given sample. Mammalian cells may contain as many as 1 x10⁵ different mRNA molecules, each of which varies in abundance within a given cell. The most abundant messages may be present at thousands of copies per cell while rare or low abundant messages may be present at less than one copy per cell. Given this large dynamic range, detection of rare and low abundant messages within the total mRNA population of a cell is difficult.

One way to solve this problem is to use large amounts of RNA in the analysis. However, this can be problematic when working with samples that have limited quantities, such as tissue biopsies, laser capture microdissection (LCM) samples, primary cell lines, and mRNA-poor cells.

Gene expression profiling using microarrays is performed using targets that are fluorescently labeled representations of cellular mRNA pools. The targets may be labeled cDNA or antisense RNA. cDNA targets are generated using a single round of reverse transcription (direct labeling). An oligo-dT primer binds to the polyA tail of mRNA and reverse transcriptase (RT) synthesizes a cDNA copy of the transcript. The input RNA can be total RNA or mRNA isolated from a cell line or tissue. Typically 5-50 µg of total RNA may be required for one experiment. However, ideally one would like to use <100 ng of total RNA for the analysis.

One method for overcoming the high RNA requirement in direct labeling is amplification. Methods for linearly amplifying mRNA to produce antisense RNA (cRNA) have been described (US patents 5,554,516, 5,716,785, 6,132,997). The input RNA can be total RNA or mRNA. mRNA is converted to a double-stranded cDNA intermediate using RT and an oligo-dT primer linked to an RNA polymerase promoter sequence. The resulting double-stranded cDNA is recognized by RNA polymerase, which then transcribes multiple copies complementary to the target sequence. Up to 1000-fold amplification can be achieved, and relatively no bias or change in the relative distribution of messages is introduced. The targets can be fluorescently labeled by incorporating dye-NMPs during the RNA synthesis reaction or by generating labeled cDNA from the amplified RNA.

One problem with both the direct and linear amplification methods is RT-catalyzed cDNA synthesis is not efficient, as mass yields of cDNA transcripts from RT-catalyzed synthesis generally do not exceed 50%. Generating one cDNA copy of every mRNA in a given population cannot be achieved due in part to the catalytic properties of RT. In addition, the quality of the mRNA template also influences how much mRNA can be converted into cDNA. When using total RNA as the input RNA, some RTs are sensitive to inhibition by ribosomal and transfer RNA. Because of these problems, more abundant messages have a higher probability of being converted to cDNA than low abundant messages. Therefore, based on statistics, as the input RNA is decreased to very small amounts, rare and low abundant messages may not be converted to cDNAs or amplified to cRNA and therefore not represented in the labeled representation of the starting mRNA population.

Detection of rare and low abundant messages is further limited by background signals stemming from high abundant messages. As the entire mRNA population is amplified, the overall number of labeled targets increases, resulting in increased background. As such, there arises a signal to noise issue: detecting a very small signal from the gene of interest within a total population of labeled targets in which there are many genes with very high signal. Additionally, the amplification that can be achieved using RNA polymerase is limited by the absolute amount of RNA generated because of product inhibition; a by-product of RNA synthesis is pyrophosphate, which in large amounts inhibits RNA polymerase.

Therefore, a method designed to limit the absolute amount of RNA generated by the RNA polymerase could have considerable value in detecting rare or low abundant messages and is of interest.

### Relevant Literature

United States Patents of interest include: 6,461,814; 6,420,105; 6,271,002; 6,268,147; 6,203,988; 6,156,502; 6,132,997; 5,932,451; 5,716,785; 5,554,516; 5,545,522; 5,437,990; 5,130,238; 6,057,134 and 5,514,545. Published U.S. Applications of interest include: 2002/0094538: 200210150917; 200210160361; and 200210119484. Antisense RNA synthesis is also discussed in Phillips and Eberwine (1996), Methods: A companion to Methods in Enzymol. 10, 283; Eberwine et al. (1992), Proc., Natl., Acad. Sci. USA 89, 3010; Eberwine (1996), Biotechniques 20, 584; and Eberwine et al. (1992), Methods in Enzymol. 216, 80.

### SUMMARY OF THE INVENTION

Methods and compositions for performing nucleic acid primer extension reactions are provided, where the subject methods produce a reduced complexity product with respect to the initial nucleic acid template.

According to a first aspect of the present invention, there is provided a method of performing a template dependent primer extension reaction as specified in claim 1.

According to a second aspect of the present invention, there is provided a method of performing a template dependent primer extension reaction as specified in claim 2.

According to a third aspect of the present invention, there is provided a kit as specified in claim 6.

According to a fourth aspect of the present invention, there is provided a kit as specified in claim 7.

According to a fifth aspect of the present invention, there is provided a method of detecting the presence of a nucleic acid analyte in a sample of nucleic acids as specified in claim 9.

### BRIEF DESCRIPTION OF THE FIGURES

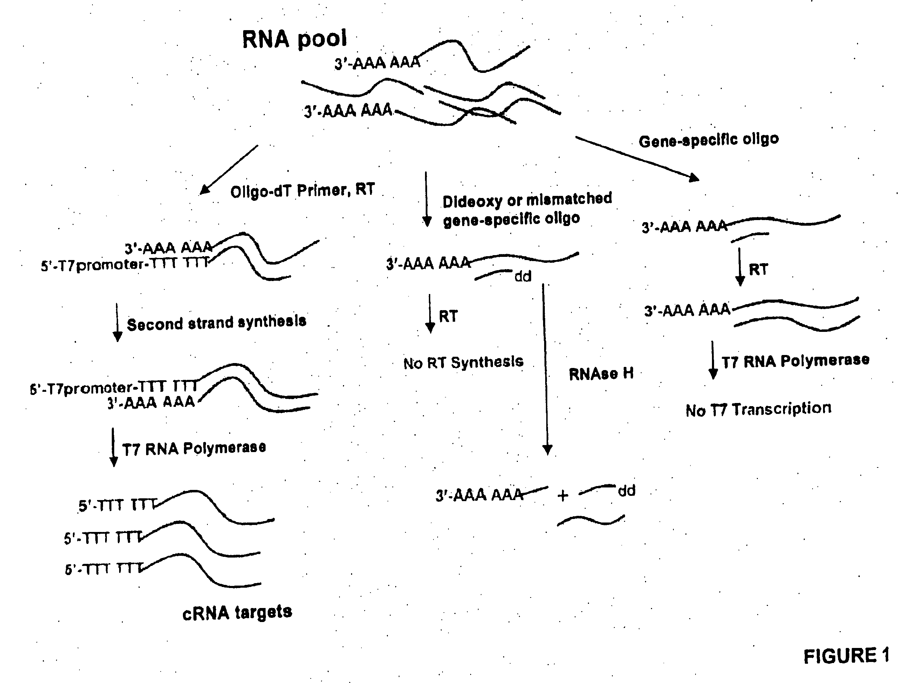

Figure 1 provides a schematic diagram of various representative embodiments of the subject methods.

### DEFINITIONS

The term "nucleic acid" as used herein means a polymer composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

A "single-stranded" nucleic acid is a nucleic acid polymer that is not paired with a different nucleic acid polymer through complementary (e.g., Watson-Crick) base pair interactions.

A "double-stranded" nucleic acid is a nucleic acid polymer that is at least partially paired with a different (complementary) nucleic acid polymer through complementary (e.g., Watson-Crick) base pair interactions. An example of a double-stranded nucleic acid is a single stranded nucleic acid that is paired with a complementary oligonucleotide. A double-stranded nucleic acid may be a DNA/DNA hybrid, an RNA/DNA hybrid, or an RNA/RNA hybrid. A DNA/RNA hybrid contains a single-stranded DNA and a complementary single-stranded RNA.

The term "oligonucleotide" as used herein denotes single stranded nucleotide multimers of from about 10 to 100 nucleotides and up to 200 nucleotides in length.

A "primer" is a single-stranded nucleic acid that can hybridize with a nucleic acid through complementary base pair interactions. A primer may hybridize with a complementary nucleic acid to result in a double-stranded nucleic acid. In certain embodiments, a primer may hybridize with a nucleic acid to result in a duplex that is substrate for primer extension. A primer suitable for primer extension may be termed an "extension primer". In other embodiments, a primer may hybridize with a nucleic acid to result in a duplex that is not a substrate for primer extension, particularly if the primer is not perfectly complementary to the nucleic acid, or if the primer is suitably modified. A primer not suitable for primer extension may be termed a "blocking primer."

The term "functionalization" as used herein relates to modification of a solid substrate to provide a plurality of functional groups on the substrate surface. By a "functionalized surface" is meant a substrate surface that has been modified so that a plurality of functional groups are present thereon.

The terms "reactive site", "reactive functional group" or "reactive group" refer to moieties on a monomer, polymer or substrate surface that may be used as the starting point in a synthetic organic process. This is contrasted to "inert" hydrophilic groups that could also be present on a substrate surface, e.g., hydrophilic sites associated with polyethylene glycol, a polyamide or the like.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids which are C-glycosides of a purine or pyrimidine base, polypeptides (proteins), polysaccharides (starches, or polysugars), and other chemical entities that contain repeating units of like chemical structure.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotides" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "oligonucleotide target element bound to a surface of a solid support" refers to an oligonucleotide or mimetic thereof, e.g., PNA, that is immobilized on a surface of a solid substrate, where the substrate can have a variety of configurations, e.g., a sheet, bead, or other structure. In certain embodiments, the collections of oligonucleotide target elements employed herein are present on a surface of the same planar support, e.g., in the form of an array.

The term "array" encompasses the term "microarray" and refers to an ordered array presented for binding to nucleic acids and the like.

An "array," includes any two-dimensional or substantially two-dimensional (as well as a three-dimensional) arrangement of addressable regions bearing nucleic acids, particularly oligonucleotides or synthetic mimetics thereof, and the like. Where the arrays are arrays of nucleic acids, the nucleic acids may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm² or even less than 10 cm², e.g., less than about 5cm², including less than about 1 cm², less than about 1 mm², e.g., 100 µm², or even smaller. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm², or even less than 50 cm², 5 cm², 1 cm² , 0.5 cm², or 0.1 cm². In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case of *in situ* fabrication, or the previously obtained nucleic acid. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

An array is "addressable" when it has multiple regions of different moieties (e.g., different oligonucleotide sequences) such that a region (i.e., a "feature" or "spot" of the array) at a particular predetermined location (i.e., an "address") on the array will detect a particular probe sequence. Array features are typically, but need not be, separated by intervening spaces. In the case of an array in the context of the present application, the "probe" will be referenced as a moiety in a mobile phase (typically fluid), to be detected by "targets" which are bound to the substrate at the various regions.

A "scan region" refers to a contiguous (preferably, rectangular) area in which the array spots or features of interest, as defined above, are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

The term "stringent assay conditions" as used herein refers to conditions that are compatible to produce binding pairs of probes and targets sufficient complementarity to provide for the desired level of specificity in the assay while being incompatible to the formation of binding pairs between binding members of insufficient complementary to provide for the desired specificity. An example of stringent assay conditions is rotating hybridization at 65°C in a salt based hybridization buffer with a total monovalent cation concentration of 1.5M (e.g., as described in U.S. Patent Application No. 09/655,482 filed on September 5, 2000, the disclosure of which is herein incorporated by reference) followed by washes of 0.5X SSC and 0.1X SSC at room temperature. Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

### DESCRIPTION OF THE SPECIFIC EMBODIMENTS

Methods and compositions for performing nucleic acid primer extension reactions that produce a reduced complexity product with respect to the initial nucleic acid template are provided. In the subject methods, template nucleic acid, e.g., RNA, is contacted with a primer composition that includes both a universal, e.g., oligo dT, primer and at least one gene specific primer under template dependent primer extension reaction conditions. The subject methods find use a variety of different applications, including the preparation of labeled nucleic acids, e.g., for use in differential gene expression analysis applications. Also provided are kits for practicing the subject methods.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are for the purpose of describing and disclosing the invention components that are described in the publications which might be used in connection with the presently described invention .

As summarized above, the present invention provides methods of performing template dependent primer extension reactions that produce reduced complexity product populations of nucleic acids with respect to their initial nucleic acid templates, as well as kits for use in practicing the subject methods. In further describing the present invention, the subject methods are discussed first in greater detail, followed by a review of representative kits for use in practicing the subject methods.

### METHODS

The subject invention provides methods for performing a template dependent primer extension reaction that results in a reduction in the amount of highly expressed transcripts as compared to an initial nucleic acid template. In other words, practice of the subject methods produces a nucleic acid product in which the most highly expressed transcripts are reduced as compared to the initial or input template nucleicacid employed in the method. This also reduces the complexity of the population by reducing the large dynamic range of individual transcripts By "complexity" is meant the variation in the abundancy of individual sequences. For example, transcript A may be present at 3000 copies per cell and a transcript B may be present at less than one copy per cell. Transcript A may represent a disproportionate amount of the total population by mass relative to transcript B. Removal of the highly abundant transcripts reduces the complexity by decreasing the magnitude of the dynamic range of the individual sequences, thereby leaving a population of sequences that are closer in abundancy and reducing the complexity so that one transcript does not represent a large fractional portion of the total population.

As summarized above, the subject methods are template dependent primer extension methods. By "template dependent primer extension method" is meant a protocol that employs an initial nucleic acid source or composition as a template in an enzymatic reaction that employs the template to direct the synthesis of a primer extension product nucleic acid, where the initial product nucleic acid in the "first round," (i.e., the immediate product) has a sequence of nucleotides that is the complement of the template that is employed to direct its synthesis. Nucleic acid template dependent primer extension reactions are well known to those in the art, and are described (among other locations) in the patents, patent applications and articles listed in the Relevant Literature section, above

In the first step of the subject methods, an initial nucleic acid template, e.g., RNA template such as an mRNA sample, is subjected to template dependent primer extension reaction conditions. While the initial nucleic acid template can be obtained from any convenient source, including both naturally occurring and synthetic sources, in many embodiments the initial nucleic acid template is obtained, e.g., derived, from a physiological source. The physiological source may be from a variety of eukaryotic sources, with eukaryotic physiological sources of interest including sources derived from single-celled organisms, such as yeast, and multicellular organisms, including plants and animals, particularly mammals, where the physiological sources from multicellular organisms may be derived from particular organs or tissues of the multicellular organism, or from isolated cells derived therefrom.

As indicated above, the nucleic acid template is, in many embodiments, an RNA template, e.g., an mRNA preparation, a total RNA preparation, etc. In obtaining the RNA template from the physiological source from which it is derived, any convenient protocol for isolation of RNA from the initial physiological source may be employed. Methods of isolating RNA from cells, tissues, organs or whole organisms are known to those of skill in the art and include those described in Maniatis et al. (1989), Molecular Cloning: A Laboratory Manual 2d Ed. (Cold Spring Harbor Press).

Following preparation of the nucleic acid template, as described above, the prepared nucleic acid template is employed in the preparation of product nucleic acids, which may be labeled product nucleic acids (as described in greater detail below) in a protocol in which a primer composition made up of both a universal primer and at least one gene specific primer is employed. In other words, the template is employed in a protocol or method in which a primer composition is characterized by including both a universal primer, e.g., a primer that is capable of hybridizing to each of the constituent nucleic acid members in the template composition, and at least one (and typically a population of) gene specific primer, e.g., a primer that has a sequence that renders it capable of hybridizing to a select one (or portion) of the total nucleic acid constituent members of the template.

The primers employed in the subject methods are typically at least about 6 nt in length, usually at least about 10 nt in length, such as about 20 nt length. The overall size of the primers may vary depending on a number of factors, e.g., the labeling protocol, etc. For example, where the methodology does not involve an amplification protocol, the primers may be oligonucleotide primers, which include at least a priming site, where such oligonucleotide primers may, in many embodiments, range in length from about 3 to about 25 nt, sometimes from about 5 to about 20 nt and sometimes from about 5 to about 10 nt. Alternatively, where the labeling protocol includes an amplification protocol, such as one that includes an in vitro transcription step (as described in greater detail below) or linear PCR amplification step, the primers may be substantially longer, e.g., where they include additional functional regions, such as amplification regions, e.g., RNA polymerase promoter sequences, linear PCR primer sequences, etc. In these types of embodiments, the primers may range in length up to about 100 nt or longer, e.g., up to about 80 nt or longer, such as up to about 70 nt or longer. In embodiments that include an amplification protocol, a universal primer typically has a priming region, e.g., oligo-dT, and an amplification region, e.g., an RNA polymerase promoter sequence or complement thereof, of a priming site for a PCR primer.

As indicated above, a feature of the subject methods is that both a universal primer and at least one gene specific primer are employed in the subject template dependent primer extension protocols. Each of these different components of the employed primer compositions is now described in greater detail separately.

As indicated above, the universal primer component of the primer compositions employed in the subject methods is a primer that is capable of hybridizing under stringent conditions to all of the different constituent template nucleic acids of the nucleic acid template. In many embodiments, the universal primer is an oligo dT primer, which includes a plurality of, e.g., at least about 6, sequential dT residues to provide for hybridization to the polyA tail of template mRNAs in the template nucleic acid preparation. Such oligo dT primers are well known to those of skill in the art.

Where the protocol being employed includes an amplification step, e.g., in vitro transcription, the universal primer may be a promoter primer that includes both a priming domain or region and an amplification domain or region. In embodiments that include amplification by *in vitro* transcription, the amplification primer employed in the template dependent primer extension reaction includes: (a) a poly-dT region for hybridization to the poly-A tail of the mRNA; and (b) an RNA polymerase promoter region 5' of the -poly-dT region that is in an orientation capable of directing transcription of antisense RNA. In other embodiments that include amplification the amplification primer contains a region that facilitates amplification, e.g., the region may be a binding site for a primer to be used in a linear PCR amplification method. In certain embodiments, the primer may be a "lock-dock" primer, in which immediately 3' of the poly-dT region is either a "G', "C", or "A" such that the primer has the configuration of 5'-TTTTTTTX.....3', where X is "G", "C", or "A". In these embodiments, the poly-dT region is sufficiently long to provide for efficient hybridization to the poly-A tail, where the region typically ranges in length from 10-50 nucleotides in length, usually 10-25 nucleotides in length, and more usually from 14 to 20 nucleotides in length.

A number of RNA polymerase promoters may be used for the amplification region of a first strand cDNA primer. Such primers are termed "promoter-primers". Suitable promoter regions will be capable of initiating transcription from an operationally linked DNA sequence in the presence of ribonucleotides and an RNA polymerase under suitable conditions. The promoter will be linked in an orientation to permit transcription of antisense RNA. A linker oligonucleotide between the promoter and the DNA may be present, and if, present, will typically comprise between about 5 and 20 bases, but may be smaller or larger as desired. The promoter region will usually comprise between about 15 and 250 nucleotides, preferably between about 17 and 60 nucleotides, from a naturally occurring RNA polymerase promoter or a consensus promoter region, as described in Alberts et al. (1989) in Molecular Biology of the Cell, 2d Ed. (Garland Publishing, Inc.). In general, prokaryotic promoters are preferred over eukaryotic promoters, and phage or virus promoters most preferred. As used herein, the term "operably linked" refers to a functional linkage between the affecting sequence (typically a promoter) and the controlled sequence (the mRNA binding site). The promoter regions that find use are regions where RNA polymerase binds tightly to the DNA and contain the start site and signal for RNA synthesis to begin. A wide variety of promoters are known and many are very well characterized. Representation promoter regions of particular interest include T7, T3 and SP6 as described in Chamberlin and Ryan, The Enzymes (ed. P. Boyer, Academic Press, New York) (1982) pp 87-108.

Where one wishes to amplify only a portion of the mRNA species in the sample, one may optionally provide for a short arbitrary sequence 3' of the poly- dT region, where the short arbitrary sequence will generally be less than 5 nucleotides in length and usually less than 2 nucleotides in length, where the dNTP immediately adjacent to the poly-dT region will not be a T residue and usually the sequence will comprise no T residue. Such short 3' arbitrary sequences are described in Ling and Pardee (1992), Science 257, 967.

Promoters primers (as well as protocols for their use) are also described in, for example, U.S. Patent Nos. 6,271,002; and 6,132,997; the disclosures of which are herein incorporated by reference.

In addition to the universal primer component, as described above, the primer compositions further include a gene specific primer component, where the gene specific primer component is made up of one or more gene specific primers, i.e., at least one gene specific primer. Gene specific primers are well known to those of skill in the art (being described in U.S. Patent Nos. 6,045,996 and 5,994,076 (among other locations)
and are primers that do not hybridize to all of the constituent members of the template, but instead hybridize to only a portion thereof, e.g., a single nucleic acid species, of the template. As such, each gene specific primer can be complementary to a sequence of nucleotides which is unique in the population of nucleic acids of the nucleic acid template, e.g. mRNAs, with which the primers are contacted, or one or more of the gene specific primers in the set may be complementary to several nucleic acids in a given population, e:g., multiple mRNAs, e.g., to conserved domains or regions found in two or more species of the template, where representative domains or regions include those comprising: repetitive sequences, such as Alu repeats, Al repeats and the like; homologous sequences in related members of a gene-family; polyadenylation signal splicing signals; or arbitrary but conversed sequences.

As indicated above, the gene specific primer component of the primer compositions employed in the subject methods includes at least one gene specific primer, where the gene specific primer component may include a plurality of distinct or different gene specific primers of differing nucleotide sequence, where by "plurality" is meant at least about 2, usually at least about 5, and more usually at least about 10, such as at least about 50, at least about 100, at least about 250, at least about 500, at least about 1000, at least about 2500, etc.

In many embodiments, a gene specific primer usually binds to a target nucleic acid species of the nucleic acid template at a site that is proximal to the binding site of the universal primer. For example; the gene specific primer and universal primer binding sites may be adjacent to each other (i.e. the 3' terminal nucleotide of the universal primer and the 5' terminal nucleotide of the gene specific primer occupy positions that are immediately adjacent to each other relative the sequence of nucleic acid species). In certain embodiments the binding sites may be separated less than about 10 (e.g., 1, 2, 3, 4, 5, 6, 7, 8, 9, or 10) bases, less than about 12 bases, less than about 15 bases, less than about 20 bases, less than about 25 bases or less than about 30 bases or more nucleotides. In other words, if the universal primer is an oligo-d(T) primer, the gene specific primer usually pairs with a position that is close in proximity, sometimes immediately proximal, to the poly(A) region.

In most embodiments, therefore, the sequence of a target nucleic acid sequence is at least partially known e.g. the polynucleotide sequence immediately preceding the poly-(A) region of an mRNA.

In principle, the gene specific primer component may include one or more gene specific primers for any nucleic acid (e.g., mRNA transcript) of interest. As such, the gene specific primer component of the primer composition can include one or more primers to specific genes that one does not wish to be represented in the final nucleic acid product. In one representative embodiment, the one or more gene specific primers directed to abundant nucleic acids present in the initial template, e.g., abundant mRNA transcripts in the template, are employed. By "abundant" is meant a nucleic acid whose copy number in the template exceeds at least about 1000 copies per cell, e.g., at least about 100 copies per cell, including at least about 10 copies per cell. As mentioned above, the nucleotide sequence of an abundant nucleic acid is usually at least partially known.

The gene specific primers of the gene specific primer component of the present invention differ from the universal primer components of the invention in a manner that provides for the preparation of nucleic acid final products (where the products may be first strand products, second strand products, transcripts therefrom, etc., depending on the particular protocol) from the universal primers but not from the gene specific primers. In other words, the gene specific primers are designed so that, depending on the particular template dependent primer extension protocol employed, e.g., whether or not the protocol includes an amplification e.g., in vitro transcription, step, final nucleic acid product is only produced from those template nucleic acids that are hybridized by the universal primer, and not the gene specific primer. In this way, the gene specific primer component serves to reduce the complexity in the final nucleic acid product, as compared to the initial template.

In certain embodiments, the template dependent primer extension reaction that is practiced is not one that includes an amplification step. Instead, the nucleic acid product is made up of nucleic acids that are the complements of the template nucleic acids and include the primers employed in the template dependent primer extension reaction. In these embodiments, the gene specific primers are modified such that they can specifically hybridize to a nucleic acid in the template collection of nucleic acids, but cannot serve as a primer for template dependent primer extension. Any convenient modification that renders the gene specific primer incapable of serving as a primer may be present in the gene specific primers of this embodiment.

In certain embodiments of this type of gene specific primer, the gene specific primers are modified so that they include a 3' mismatch, in other words, one or more mismatched nucleotides located at their 3' ends. In other words, they include one or more mismatch nucleotides at their 3' ends, where a mismatch is not the complement of the corresponding template nucleotide when the primer is hybridized to the template. The one or more mismatches may be located any of positions 1, 2 or 3 relative to the 3' end. In certain embodiments, the mismatch is the 3' terminal nucleotide.

In yet other embodiments of this type, the gene specific primer is one that includes a 3' dideoxy nucleotide. In other words, the 3' terminal nucleotide of the gene specific primer is a dideoxy nucleotide that tacks the 3' OH moiety necessary to initiate synthesis in a template dependent primer extension reaction.

In embodiments where the template dependent primer extension protocol includes an amplification step, e.g., where the universal primer component is made up of amplification primers, as described above, the gene specific primers may not be modified, as described above, but instead differ from the universal primers in that they lack the domain necessary for amplification, e.g., in vitro transcription. In this manner, final nucleic acid product is generated in the protocol only from the universally primed templates, and not the gene specific primed templates.

The primers described above and throughout this specification may be prepared using any suitable method, such as, for example, the known phosphotriester and phosphite triester methods, or automated embodiments thereof. In one such automated embodiment, dialkyl phosphoramidites are used as starting materials and may be synthesized as described by Beaucage et al. (1981), Tetrahedron Letters 22, 1859. One method for synthesizing oligonucleotides on a modified solid support is described in U.S. Patent No. 4,458,066, the disclosure of which is herein incorporated by reference. It is also possible to use a primer that has been isolated from a biological source (such as a restriction endonuclease digest). The primers herein are selected to be "substantially" complementary to each specific sequence to be amplified, *i.e.*; the primers should be sufficiently complementary to hybridize to their respective targets. Therefore, the primer sequence need not reflect the exact sequence of the target, and can, in fact be "degenerate." Non-comptementary bases or longer sequences can be interspersed into the primer, provided that the primer sequence has sufficient complementarity with the sequence of the target to be amplified to permit hybridization and extension.

In the first step of the subject methods (as summarized above), the primer compositions (made up of both the universal and gene specific components, as described above) are employed in a template dependent primer extension reaction, e.g., by subjecting the combined template and primer composition to primer extension reaction conditions. As such, the primer composition is contacted with the nucleic acid template under conditions sufficient for a primer extension reaction to occur. In certain embodiments, the template dependent primer extension reaction only includes a primer extension step, e.g., as may be practiced in a protocol that does not include nucleic acid amplification. In yet other embodiments, the template dependent primer extension reaction protocol includes a primer extension step, as well as one or more additional steps, such as a conversion step from an RNA/DNA hybrid to double-stranded cDNA, transcription step, etc., as may be found in linear amplification protocols.

In those embodiments where the template dependent primer extension reaction only includes a primer extension step, the primer composition and template are combined and maintained with reagents necessary for primer extension to occur, e.g., a reverse transcriptase activity, such as those derived from Moloney murine leukemia virus (MMLV-RT), avian myeiobiasiosis virus (AMV-RT), bovine leukemia virus (BLV-RT), Rous sarcoma virus (RSV) and human immunodeficiency virus (HIV-RT), including RNase H minus reverse transcriptases; deoxyribonucleotides (dGTP, dATP, dCTP, dTTP); additional reagents which include, but are not limited to: dNTPs; monovalent and divalent cations, *e.g*. KCI, MgCl₂; sulfhydryl reagents, *e.g*. dithiothreitol; and buffering agents, *e.g.* Tris-Cl; etc., as is known in the art. The reaction mix is maintained for a period of time and at a temperature sufficient for the primer extension reaction to occur, where such conditions are known to those of skill in the art.

In those embodiments where the particular template dependent primer extension reaction includes a primer extension step and one or more additional steps, e.g., amplification protocols, the template dependent primer extension reaction conditions to which the template and primer are subjected are typically conditions that ultimately result in the production of double stranded cDNA products, which resultant products may or may not ultimately be intermediate products, e.g., where the protocol includes an in vitro transcription step. In these embodiments, the primers of the primer composition are hybridized with a sufficient amount of an initial nucleic acid template, e.g., mRNA, total RNA, etc., and the resultant primer-nucleic acid (e.g., mRNA) hybrids are converted to double-stranded cDNA products.

The primer composition is contacted with the template nucleic acids of under conditions that allow the primers to hybridize to the template nucleic acids in the sample, e.g., the poly dT portions of the universal primers to hybridize to the polyA tails of mRNAs in the template and the gene specific primers to specifically hybridize to complementary mRNAs present in the template. The resultant duplexes are then maintained under conditions sufficient to produce double- stranded cDNA from the duplexes. As such, the resultant duplexes are maintained in the presence of reagents necessary to, and for a period of time sufficient to, convert the primer-mRNA hybrids to double stranded cDNAs.

The catalytic activities required to convert primer-mRNA hybrid to double-stranded cDNA are an RNA-dependent DNA polymerase activity, a RNaseH activity, and a DNA-dependent DNA polymerase activity. Most reverse transcriptases, including those derived from Moloney murine leukemia virus (MMLV-RT), avian myeloblastosis virus (AMV-RT), bovine leukemia virus (BLV-RT), Rous sarcoma virus (RSV) and human immunodeficiency virus (HIV-RT) catalyze each of these activities. These reverse transcriptases are sufficient to convert primer-mRNA hybrid to double-stranded DNA in the presence of additional reagents which include, but are not limited to: dNTPs; monovalent and divalent cations, *e.g*. KCI, MgCl₂; sulfhydryl reagents, *e.g*. dithiothreitol; and buffering agents, *e.g.* Tris-Cl. Alternatively, a variety of proteins that catalyze one or two of these activities can be added to the cDNA synthesis reaction. For example, MMLV reverse transcriptase lacking RNaseH activity (described in U.S. Patent No. 5,405,776) which catalyzes RNA-dependent DNA polymerase activity and DNA-dependent DNA polymerase activity, can be added with a source of RNaseH activity, such as the RNaseH purified from cellular sources, including *Escherichia coli.* These proteins may be added together during a single reaction step, or added sequentially during two or more substeps. Finally, additional proteins that may enhance the yield of double-stranded DNA products may also be added to the cDNA synthesis reaction. These proteins include a variety of DNA polymerases (such as those derived from *E. coli*, thermophilic bacteria, archaebacteria, phage, yeasts, Neurosporas, Drosophilas, primates and rodents), and DNA Ligases (such as those derived from phage or cellular sources, including T4 DNA Ligase and *E. coli* DNA Ligase).

In certain embodiments, it is desirable to include one or more detergents, where the detergent enhances the amount of aRNA that is ultimately produced. If a detergent is employed a number of detergent types are useful. Detergents such as bile salts, cholate, deoxycholate, lithocholate may be used. Also useful are ionic detergents such as anionic, cationic or zwitterionic detergents. Guanidine salts, such as Guanidine hydrochloride and Guanidine thiocyanate may be used. Additionally, nonionic surfactants such as polyoxyethylene sorbitol ester or polyoxyethylene p-t octylphenol may be used. Specifically the Tween series, including Tween 20 and Tween 80, and Triton series, including Triton N-101 and NP-40, are preferred. Tween-80 or Triton X-100 are most preferred. While the concentration employed may vary, in many embodiments the concentration ranges from about 0.001% to about 0.1%, and often from about 0.005% to about 0.025 %.

Conversion of primer-mRNA hybrids to double-stranded cDNA by reverse transcriptase proceeds through an RNA:DNA intermediate which is formed by extension of the hybridized promoter-primer by the RNA-dependent DNA polymerase activity of reverse transcriptase. The RNaseH activity of the reverse transcriptase then hydrolyzes at least a portion of the RNA:DNA hybrid, leaving behind RNA fragments that can serve as primers for second strand synthesis (Meyers et al., Proc. Nat'l Acad. Sci. USA (1980) 77:1316 and Olsen & Watson, Biochem. Biophys. Res. Commun. (1980) 97:1376). Extension of these primers by the DNA-dependent DNA polymerase activity of reverse transcriptase results in the synthesis of double-stranded cDNA. Other mechanisms for priming of second strand synthesis may also occur, including "self-priming" by a hairpin loop formed at the 3' terminus of the first strand cDNA (Efstratiadis et al. (1976), Cell 7, 279; Higuchi et al. (1976), Proc. Natl, Acad, Sci USA 73, 3146; Maniatis et al. (1976), Cell 8, 163; and Rougeon and Mach (1976), Proc. Natl. Acad. Sci. USA 73, 3418; and "non-specific priming" by other DNA molecules in the reaction, *i.e.* the promoter-primer.

The second strand cDNA synthesis results in the creation of a double-stranded promoter region. The second strand cDNA includes.not only a sequence of nucleotide residues that comprise a DNA copy of the mRNA template, but also additional sequences at its 3' end which are complementary to the promoter-primer used to prime first strand cDNA synthesis. The double-stranded promoter region serves as a recognition site and transcription initiation site for RNA polymerase, which uses the second strand cDNA as a template for multiple rounds of RNA synthesis during the next stage of the subject methods.

Depending on the particular protocol, the same or different DNA polymerases may be employed during the cDNA synthesis step. For example, a single reverse transcriptase, most preferably MMLV-RT, may be used as a source of all the requisite activities necessary to convert primer-mRNA hybrid to double-stranded cDNA. Alternatively, the polymerase employed in first strand cDNA synthesis may be different from that which is employed in second strand cDNA synthesis. Specifically, a reverse transcriptase lacking RNaseH activity (e.g. Superscript II^{™}) may be combined with the primer-mRNA hybrid during a first substep for first strand synthesis. A source of RNaseH activity, such as *E. coli* RNaseH or MMLV-RT, may be added during a second substep to initiate second strand synthesis. In yet other embodiments, the requisite activities are provided by a plurality of distinct enzymes. The manner is which double-stranded cDNA is produced from the initial mRNA is not critical to certain embodiments of the invention. However, in certain embodiments one employs MMLV-RT, or a combination of Superscript II^{™} and MMLV-RT, or a combination of Superscript II™ and *E*. *coli* RNaseH, for cDNA synthesis as these embodiments yield certain desired results.

In those embodiments where an amplification step is included, (e.g., where the universal primer includes an RNA polymerase promoter and the gene specific primers do not), the next step of the subject method may be the preparation of antisense RNA from the double-stranded cDNA prepared in the first step. During this step, the double-stranded cDNA produced in the first step is transcribed by RNA polymerase to yield antisense RNA, which is complementary to the initial mRNA target from which it is amplified.

Depending on the particular protocol employed, the subject methods may or may not include a step in which the double-stranded cDNAs produced as described above are physically separated from the reverse transcriptase employed in the cDNA production step prior to the transcription step. As such, in certain embodiments, the cDNAs produced in the first step of the subject methods are separated from the reverse transcriptase employed in this first step prior to the second transcription step described in greater detail below. In these embodiments; any convenient separation protocol may be employed, including the phenol/chloroform extraction and ethanol precipitation (or dialysis), protocol as described in U.S. Patent Nos. 5,554,516 and U.S. Patent No. 5,716,785, the disclosures of which are herein incorporated by reference.

The subject methods may be adapted for use in a variety of nucleic acid amplification procedures, including linear PCR amplification, non-linear PCR amplification and amplification using polymerases other than T7, T3 and SP6. Such methods are generally described in Ausubel, et al, (Short Protocols in Molecular Biology, 3rd ed., Wiley & Sons, 1995) and Sambrook, et al, (Molecular Cloning: A Laboratory Manual, Third Edition, 2001 Cold Spring Harbor, N.Y.). Several commercially available kits may be adapted to be perform the subject methods, including MESSAGEAMP™ (Ambion, Austin, TX), RIBOAMP ™ (Arcturus Inc., Mountain View, CA) and the BD Atlas SMART ™ Fluorescent Probe Amplification Kit (Clontech, Palo Alto, CA). In many embodiments, these kits may be adapted for use in the subject methods by the addition of at least one gene specific primer, as described above.

In yet other embodiments, the subject methods do not involve a step in which the double-stranded cDNA is physically separated from the reverse transcriptase following double-stranded cDNA preparation. In these embodiments, the reverse transcriptase that is present during the transcription step is rendered inactive. Thus, the transcription step is carried out in the presence of a reverse transcriptase that is unable to catalyze RNA-dependent DNA polymerase activity, at least for the duration of the transcription step. As a result, the antisense RNA products of the transcription reaction cannot serve as substrates for additional rounds of amplification and the amplification process cannot proceed exponentially.

The reverse transcriptase present during the transcription step may be rendered inactive using any convenient protocol, including those described in U.S. Patent No. 6,132,997; the disclosure of which is herein incorporated by reference. As described in this reference, the transcriptase may be irreversibly or reversibly rendered inactive. Where the transcriptase is reversibly rendered inactive, the transcriptase is physically or chemically altered so as to no longer able to catalyze RNA-dependent DNA polymerase activity. The transcriptase may be irreversibly inactivated by any convenient means. Thus, the reverse transcriptase may be heat inactivated, in which the reaction mixture is subjected to heating to a temperature sufficient to inactivate the reverse transcriptase prior to commencement of the transcription step. In these embodiments, the temperature of the reaction mixture and therefore the reverse transcriptase present therein is typically raised to 55°C to 70 °C for 5 to 60 minutes, usually to about 65 °C for 15 to 20 minutes. Alternatively, reverse transcriptase may irreversibly inactivated by introducing a reagent into the reaction mixture that chemically alters the protein so that it no longer has RNA-dependent DNA polymerase activity. In yet other embodiments, the reverse transcriptase is reversibly inactivated. In these embodiments, the transcription may be carried out in the presence of an inhibitor of RNA-dependent DNA polymerase activity. Any convenient reverse transcriptase inhibitor may be employed which is capable of inhibiting RNA-dependent DNA polymerase activity a sufficient amount to provide for linear amplification. However, these inhibitors should not adversely affect RNA polymerase activity. Reverse transcriptase inhibitors of interest include ddNTPs, such as ddATP, ddCTP, ddGTP or ddTTP, or a combination thereof, the total concentration of the inhibitor typically ranges from about 50 µM to 200 µM.

Regardless of whether the cDNA is separated from the reverse tanscriptase prior to the transcription step, for the transcription step, the presence of the RNA polymerase promoter region on the double-stranded cDNA is exploited for the production of antisense RNA. To synthesize the antisense RNA, the double-stranded DNA is contacted with the appropriate RNA polymerase in the presence of the four ribonucleotides, under conditions sufficient for RNA transcription to occur, where the particular polymerase employed will be chosen based on the promoter region present in the double-stranded DNA, *e.g.* T7 RNA polymerase, T3 or SP6 RNA polymerases, *E. coli* RNA polymerase, and the like. Suitable conditions for RNA transcription using RNA polymerases are known in the art, see *e.g.* Milligan and Uhlenbeck (1989), Methods in Enzymol. 180, 51.

A feature of the above amplification embodiments is that small amounts of template nucleic acid may be employed. In these embodiments, amount of initial template, e.g., total RNA, sample that is employed may vary, and may be as low as 6 µg or lower, e.g., about 1 µg or lower; about 500 ng or lower, about 100 ng or lower, etc., where the amount in many embodiments ranges from about 100 ng to about 10 µg, usually from about 500 ng to about 6µg, and the amount of total RNA sample employed in certain embodiments does not exceed about 20 µg, and often does not exceed about 10µg.

### UTILITY

The resultant nucleic acid products of the subject methods finds use in a variety of applications. For example, the resultant nucleic acid products, e.g., first strand cDNAs, antisense RNAs, etc., can be used in expression profiling analysis on such platforms as DNA microarrays, for construction of "driver" for subtractive hybridization assays, for cDNA library construction, and the like.

Especially facilitated by the subject methods are studies of differential gene expression in mammalian cells or cell populations. The cells may be from blood (*e.g.*, white cells, such as T or B cells) or from tissue derived from solid organs, such as brain, spleen, bone, heart, vascular, lung, kidney, liver, pituitary, endocrine glands, lymph node, dispersed primary cells, tumor cells, or the like.

Depending on the particular intended use of the subject methods, the nucleic acid products may be labeled. One way of labeling which may find use in the subject invention is isotopic labeling, in which one or more of the nucleotides is labeled with a radioactive label, such as ³²S, ³²P, ³H, or the like. Another means of labeling is fluorescent labeling in which fluorescently tagged nucleotides, e.g. CTP, are incorporated into the antisense RNA product during the transcription step. Fluorescent moieties which may be used to tag nucleotides for producing labeled antisense RNA include: fluorescein, the cyanine dyes, such as Cy3, Cy5, Alexa 555, Bodipy 630/650, and the like. Other labels may also be employed as are known in the art.

Means for labeling nucleic acids are generally well known in the art (e.g. Brumbaugh et al Proc Natl Acad Sci U S A 85, 5610-4, 1988; Hughes et al. Nat Biotechnol 19, 342-7, 2001, Eberwine et al Biotechniques. 20:584-91, 1996, Ausubel, et al, Short Protocols in Molecular Biology, 3rd ed., Wiley & Sons, 1995 Sambrook, et al, Molecular Cloning: A Laboratory Manual, Third Edition, 2001 Cold Spring Harbor, N.Y. and DeRisi et al. Science 278:680-686, 1997) and usually involve either chemical modification of a nucleic acid (indirect labeling), or labeled nucleotide that is incorporated into a nucleic acid by nucleic acid replication, e.g., using a polymerase (direct labeling).

Chemical modification methods for labeling a nucleic acid sample usually include incorporation of a reactive nucleotide into a nucleic acid, e.g., an amine-allyl nucleotide derivative such as 5-(3-aminoallyl)-2'-deoxyuridine 5'-triphosphate, during first strand or second strand cDNA synthesis, or during nucleic acid amplification, followed by chemical conjugation of the reactive nucleotide to a label, e.g. a N-hydroxysuccinimdyl of a label such as Cy-3 or Cy5 to make a labeled nucleic acids (Brumbaugh et al Proc Natl Acad Sci U S A 85, 5610-4, 1988 and Hughes et al. Nat Biotechnol 19, 342-7, 2001).

Suitable labels may also be incorporated into a sample by means of nucleic acid replication (e.g. during first strand or second strand cDNA synthesis, or nucleic acid amplification) where modified nucleotides such as modified deoxynucleotides, ribonucleotides, dideoxynucleotides, etc., or closely related analogues thereof, e.g. a deaza analogue thereof, in which a moiety of the nucleotide, typically the base, has been modified to be bonded to the label. Modified nucleotides are incorporated into a nucleic acid by the actions of a nucleic acid-dependent DNA or RNA polymerases, and a copy of the nucleic acid in the sample is produced that contains the label. Methods of labeling nucleic acids by a variety of methods, e.g., random priming, nick translation, RNA polymerase transcription, etc., are well generally known in the art (see, e.g., Ausubel, et al, Short Protocols in Molecular Biology, 3rd ed., Wiley & Sons, 1995 Sambrook, et al, Molecular Cloning: A Laboratory Manual, Third Edition, 2001 Cold Spring Harbor, N.Y. and DeRisi et al. Science 278:680-686, 1997).

As such, the subject methods of nucleic acid generation find use in nucleic acid analyte detection applications, where the subject methods are employed to generate the nucleic acid analyte. Specific analyte detection applications of interest include hybridization assays in which the nucleic acid produced by the subject methods are hybridized to arrays of probe nucleic acids.

An "array", unless a contrary intention appears, includes any one-, two-or three-dimensional arrangement of addressable regions bearing a particular chemical moiety or moieties (for example, biopolymers such as polynucleotide sequences) associated with that region. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other). An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

In these assays, a sample of target nucleic acids is first prepared according to the methods described above, where preparation may include labeling of the target nucleic acids with a label, e.g. a member of signal producing system. Following sample preparation, the sample is contacted with an array under hybridization conditions, whereby complexes are formed between target nucleic acids that are complementary to probe sequences attached to the array surface. The presence of hybridized complexes is then detected. Specific hybridization assays of interest which may be practiced using the subject arrays include: gene discovery assays, differential gene expression analysis assays; nucleic acid sequencing assays, and the like. Patents and patent applications describing methods of using arrays in various applications include: 5,143,854; 5,288,644; 5,324,633; 5,432,049; 5,470,710; 5,492,806; 5,503,980; 5,510,270; 5,525,464; 5,547,839; 5,580,732; .. 5,661,028; 5,800,992; the disclosures of which are herein incorporated by reference.

In certain embodiments, the subject methods include a step of transmitting data from at least one of the detecting and deriving steps, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As.such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc

As such, in using an array made by the method of the present invention, the array will typically be exposed to a sample (for example, a fluorescently labeled analyte, e.g., protein containing sample) and the array then read. Reading of the array may be accomplished by illuminating the array and reading the location and intensity of resulting fluorescence at each feature of the array to detect any binding complexes on the surface of the array. For example, a scanner may be used for this purpose which is similar to the AGILENT MICROARRAY SCANNER scanner available from Agilent Technologies, Palo Alto, CA. U.S. Patents describing suitable scanner devices and methods for their use in the reading of arrays include, but are not limited to: 5,585,639; 5,763,870; 6,084,991; 6,222,664; 6,284,465; 6,371,370; 6,320,196; and 6,406,849, the disclosures of which are herein incorporated by reference.

However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). Results from the reading may be raw results. (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample). The results of the reading (processed or not) may be forwarded (such as by communication) to a remote location if desired, and received there for further use (such as further processing).

### KITS

Also provided are kits for use in the subject invention, where such kits may include containers, each with one or more of the various reagents (typically in concentrated form) utilized in the methods. Present in the kits is at least a primer composition made up of both gene specific and universal primers, as described above, where these two components may or may not be present in combined form (e.g., as a mixture) in the kits. The kits also typically include additional reagents that find use in the subject methods, e.g., buffers, the appropriate nucleotide triphosphates (*e.g.* dATP, dCTP, dGTP, dTTP, ATP, CTP, GTP and UTP), reverse transcriptase, RNA polymerase, and the promoter-primer of the present invention. Also present in the kits may be total RNA isolation reagents, e.g., RNA extraction buffer, proteinase digestion buffer; proteinase K, etc. Also present in the kits may be one or more detergents.

Finally, the kits may further include instructions for using the kit components in the subject methods. The instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g., CD-ROM, diskette, etc.

The following examples are offered by way of illustration and not by way of limitation.

### EXPERIMENTAL

Example 1: Total RNA is extracted from HeLa cells using traditional methods (eg Trizol, Qiagen). Two labeling reactions are carried out as described below, with one sample labeled using Cyanine 3 and the second one using Cyanine 5. A solution containing 10 µg of total RNA is transferred to one microfuge tube containing 100 pmol oligo dT primer and to a second tube containing 100 pmol oligo dT and 10 pmol of the gene specific primer to GAPDH (5'-GGTTGAGCACAGGGTACT-3') (SEQ ID NO:01). The gene specific primer contains dideoxythymidylate at the 3' position, which prevents the DNA polymerse from extending from the 3' terminal end of the primer. An annealing reaction is performed by heating the reaction to 70°C for 10 min. and then transferring the tube to ice for 5 min. Reaction components are added to a final concentration of 100 µM dNTPs (dATP, TTP, dGTP), 25 µM dCTP, 50 mM Tris-HCl, pH 8.3, 75 mM KCI, 3 mM MgCl₂, and 10 mM DTT. Cyanine 3- dCTP (25 µM final) is added to the sample containing the gene specific primer and Cyanine 5-dCTP (25 µM final) is added to the sample containing only the oligo dT primer. The reaction is initiated by the addition of 200 U MMLV RT, and incubated at 42°C in a waterbath for 60 min. The RT is heat inactivated by incubation at 70°C for 10 min., and 0.05 mg RNAse A is added to degrade the RNA. The two samples are pooled and purified using the Qiagen Qiaquick PCR kit, following the method described in the Agilent Direct Label Kit User's PCR kit, according to the Agilent Direct Label Kit User's manual. The labeled products are denatured at 95°C for 3 minutes, cooled to room temperature, and diluted into Agilent's 2x in situ Hybridization buffer (5184-3568). The solution is loaded onto an Agilent Human 1A oligo microarray (G4140A) and allowed to hybridize overnight at 60°C, as described in the Agilent Oligo Microarray User's manual. The array is washed, scanned, and feature extracted according to the manufacturer's instructions. The probe on the microarray that binds to GAPDH is expected to give a log ratio of 2 (log ratio of Cyanine 5/Cyanine 3). The Cyanine 3 labeled sample should have minimal fluorescent signal representing the GAPDH cDNA, whereas the Cyanine 5 labeled sample should have a very large fluorescent signal representing the GAPDH cDNA.

Example 2: Total RNA is extracted from HeLa cells using traditional methods (eg Trizol, Qiagen). Two labeling reactions are carried out as described below, with one sample labeled using Cyanine 3 and the second one using Cyanine 5. A solution containing 1 µg of total RNA is transferred to one microfuge tube containing 30 pmol oligo. dT-T7 promoter primer and to a second tube containing 30 pmol oligo dT-T7 promoter primer and 3 pmol of the gene specific primer to GAPDH (5'-GGTTGAGCACAGGGTACT-3') (SEQ ID NO:01). The gene specific primer contains a dideoxythymidylate at the 3' position, which prevents the DNA polymerse from extending from the 3' terminal end of the primer. An annealing reaction is performed by heating the reaction to 70°C for 10 min. and then transferring the tube to ice for 5 min. Reaction components are added to a final concentration of 500 µM dNTPs (dATP, TTP, dGTP, dCTP), 50 mM Tris-HCl, pH 8.3, 75 mM KCl, 3mM MgCl₂, 0.015% Triton X-100, and 10 mM DTT. The reaction is initiated by the addition of 200 U MMLV RT, and incubated at 42°C in a waterbath for 120 min. The RT is heat inactivated by heating to 70°C for 10 min., and the reaction tubes are put on ice for 5 min.

A T7 RNA transcription is next performed. Cyanine 3-CTP (300 µM final) is added to the reaction containing the gene specific primer and Cyanine 5-CTP (300 µM) is added to the reaction containing on the oligo dT-T7 promoter primer. The following components are added to each reaction to a final concentration of 52 mM Tris-HCl, pH 8.0, 15 mM MgCl₂, 19 mM KCI, 25 mM NaCl, 2 mM spermidine, 10 mM DTT, 2.5 mM each ATP, GTP, UTP, 0.75 mM CTP, 2000 U T7 RNA polymerase, 18 U RNaseOUT, 0.12 U Inorganic pyrophosphatase, 4% PEG. Transcription is carried out at 40°C for 120 minutes. The cRNA is purified using Qiagen RNeasy Mini column, per manufacturer's instructions.

1 µg of each Cyanine 3- and Cyanine 5-labeled cRNA is combined and diluted into Agilent's 2x in situ Hybridization buffer (5184-3568). The solution is loaded onto an Agilent Human 1A oligo microarray (G4140A) and allowed to hybridize overnight at 60°C, as described in the Agilent Oligo Microarray User's manual. The array is washed, scanned, and feature extracted according to the manufacturer's instructions. The probe on the microarray that binds to GAPDH is expected to give a log ratio of 2 (log ratio of Cyanine 5/Cyanine 3). The Cyanine 3 labeled sample should have minimal fluorescent signal representing the GAPDH cDNA, whereas the Cyanine 5 labeled sample should have a very large fluorescent signal representing the GAPDH cDNA

The above results and discussion demonstrate that novel and improved methods of producing primer extension or primer extension derived products, e.g., for use in gene expression analysis applications, are provided. Advantages of the subject methods include, but are not limited to: (a) the ability to enrich for rare and low abundant messages within a population of highly expressed messages without having to purify the RNA, where this advantage is achieved by selectively inhibiting highly expressed messages; (b) the ability to use low amounts, e.g., 100 ng or less total RNA, of inputs nucleic acid template without experiencing a concomitant loss of low abundant messages resulting from signal to noise consequences; (c) the ability to increase detection sensitivity of low abundant messages in the background of highly abundant messages by selective reduction of the highly abundant subpopulation; (d) the ability to enrich for low abundant messages by pretreating the template with inhibitor gene specific primers to remove polyA tails before isolation of mRNA from the initial source; and (e) the ability to avoid the problem of product inhibition that limits the total amount of cRNA generated by reducing the population of unwanted products. As such, the subject methods represent a significant contribution to the art.

## Claims

1. A method of performing a template dependent primer extension reaction, said method comprising:
contacting an initial RNA template
with:
(a) a primer composition comprising both a universal primer that includes a plurality of sequential dT residues to provide for hybridization to the polyA tail of template mRNAs, and at least one RNA-binding gene specific primer having a sequence that renders it capable of hybridizing to only a select one or portion of the template mRNAs, wherein the gene specific primer includes one or more mismatch nucleotides at its 3' end or wherein the gene specific primer includes a 3' dideoxy nucleotide, and
(b) a reverse transcriptase activity,
under primer extension reaction conditions to perform said template dependent primer extension reaction.

2. A method of performing a template dependent primer extension reaction, said method comprising:
(i) contacting an initial RNA template with a primer composition comprising both a universal promoter primer that includes a poly-dT region for hybridization to the poly-A tail of template mRNAs and an RNA polymerase promoter region 5' of the poly-dT region, and at least one RNA-binding gene specific primer having a sequence that renders it capable of hybridizing to only a select one or portion of the template mRNAs and which lacks an RNA polymerase promoter region, to produce primer-mRNA hybrids;
(ii) converting the primer-mRNA hybrids to double stranded cDNA products by contacting the primer-mRNA hybrids with an RNA-dependent DNA polymerase activity, a RNaseH activity and a DNA-dependent DNA polymerase activity; and
(iii) transcribing the double-stranded cDNA products containing a double-stranded RNA polymerase promoter region using an RNA polymerase to yield antisense RNA.

3. A method as claimed in claim 2, wherein the gene specific primer cannot serve as a primer for template dependent primer extension.

4. A method as claimed in claim 1, 2 or 3, wherein said universal primer and said RNA-binding gene specific primer are complementary to positions of a single RNA species that are separated by less than 15 nucleotides.

5. A method according to any preceding claim, wherein said primer composition comprises a plurality of gene specific primers.

6. A kit for use in practising a method as claimed in claim 1, said kit comprising:
(a) a universal primer that includes a plurality of sequential dT residues to provide for hybridization to the polyA tail of template mRNAs; and
(b) at least one gene specific primer having a sequence that renders it capable of hybridizing to only a select one or portion of the template mRNAs, wherein the gene specific primer includes a 3' dideoxy nucleotide.

7. A kit for use in practising a method as claimed in claim 2, said kit comprising:
(a) a universal promoter primer that includes (i) a poly-dT region for hybridization to the poly-A tail of template mRNAs, and (ii) an RNA polymerase promoter region 5' of the poly-dT region; and
(b) at least one gene specific primer having a sequence that renders it capable of hybridizing to only a select one or portion of the template mRNAs and which lacks an RNA polymerase promoter region.

8. A kit as claimed in claim 6 or 7, wherein said kit further comprises at least one polymerase.

9. A method of detecting the presence of a nucleic acid analyte in a sample of nucleic acids produced from an initial RNA template by means of a method as claimed in claim 1 or 2, said method comprising:
(a) producing said sample of nucleic acids by means of a method as claimed in any of claims 1 to 5;
(b) contacting said sample with a nucleic acid array;
(c) detecting any binding complexes on the surface of the said array to obtain binding complex data; and
(d) determining the presence of said nucleic acid analyte in said sample using said binding complex data.

## Patentansprüche

1. Verfahren zum Durchführen einer matrixabhängigen Primer-Verlängerungsreaktion, wobei das Verfahren aufweist:
eine RNS-Startmatrix in Kontakt bringen mit:
(a) einer Primer-Zusammensetzung, die sowohl einen Universal-Primer mit einer Vielzahl sequenzieller dT-Reste zum Hybridisieren mit dem Poly-A-Ende von mRNS-Matrizen als auch mindestens einen genspezifischen RNS-bindenden Primer mit einer Sequenz beinhaltet, welche diesen zur Hybridisierung mit nur einer ausgewählten mRNS-Matrix oder einem Teil davon befähigt, wobei der genspezifische Primer ein oder mehrere falsche Nucleotide an seinem 3'-Ende oder wobei der genspezifische Primer ein 3'-Dideoxy-Nucleotid beinhaltet, und
(b) einer Reverse-Transkriptase-Aktivität, um unter den Reaktionsbedingungen der Primer-Verlängerung die matrixabhängige Primer-Verlängerungsreaktion durchzuführen.

2. Verfahren zum Durchführen einer matrixabhängigen Primer-Verlängerungsreaktion, wobei das Verfahren die folgenden Schritte aufweist:
(i) eine RNS-Startmatrix mit einer Primer-Zusammensetzung in Kontakt bringen, die sowohl einen Universal-Promotor-Primer mit einem Poly-dT-Bereich zum Hybridisieren mit dem Poly-A-Ende von mRNS-Matrizen und einen RNS-Polymerase-Promotorbereich 5' des Poly-dT-Bereichs als auch mindestens einen genspezifischen RNS-bindenden Primer mit einer Sequenz aufweist, die ihn zum Hybridisieren mit nur einer ausgewählten mRNS-Matrix oder einem Teil davon befähigt und welchem ein RNS-Polymerase-Promotorbereich fehlt, um Primer-mRNS-Hybride zu erzeugen;
(ii) Umwandeln der Primer-mRNS-Hybride in zweisträngige cDNS-Produkte durch Zusammenbringen der Primer-mRNS-Hybride mit einer RNS-abhängigen DNS-Polymerase-Aktivität, einer RNaseH-Aktivität und einer DNS-abhängigen DNS-Polymerase-Aktivität; und
(iii) Transkribieren der einen zweisträngigen RNS-Polymerase-Promotorbereich enthaltenden zweisträngigen cDNS-Produkte unter Verwendung einer RNS-Polymerase, um komplementäre RNS zu erzeugen.

3. Verfahren nach Anspruch 2, wobei der genspezifische Primer nicht als Primer für die matrixabhängige Primer-Verlängerung dienen kann.

4. Verfahren nach Anspruch 1, 2 oder 3, wobei der Universal-Primer und der genspezifische RNS-bindende Primer zu Positionen eines RNS-Einzelmoleküls komplementär sind, die durch weniger als 15 Nucleotide voneinander getrennt sind.

5. Verfahren nach einem der vorhergehenden Ansprüche, wobei die Primer-Zusammensetzung eine Vielzahl von genspezifischen Primern aufweist.

6. Chemikaliensatz zum Durchführen eines Verfahrens nach Anspruch 1, wobei der Chemikaliensatz aufweist:
(a) einen Universal-Primer mit einer Vielzahl sequenzieller dT-Reste, um die Hybridisierung mit dem Poly-A-Ende von mRNS-Matrizen zu ermöglichen; und
(b) mindestens einen genspezifischen Primer mit einer Sequenz, die ihn zur Hybridisierung mit nur einer ausgewählten mRNS-Matrix oder einem Teil davon befähigt, wobei der genspezifische Primer ein 3'-Dideoxy-Nucleotid beinhaltet.

7. Chemikaliensatz zum Durchführen eines Verfahrens nach Anspruch 2, wobei der Chemikaliensatz aufweist:
(a) einen Universal-Promotor-Primer mit (i) einem Poly-dT-Bereich zum Hybridisieren mit dem Poly-A-Ende von mRNS-Matrizen und (ii) einem RNS-Polymerase-Promotorbereich 5' des Poly-dT-Bereichs; und
(b) mindestens einen genspezifischen Primer mit einer Sequenz, die ihn zum Hybridisieren mit nur einer ausgewählten mRNS-Matrix oder einem Teil davon befähigt und welchem ein RNS-Polymerase-Promotorbereich fehlt.

8. Chemikaliensatz nach Anspruch 6 oder 7, wobei der Chemikaliensatz ferner mindestens eine Polymerase aufweist.

9. Verfahren zum Detektieren eines Nucleinsäureanalyten in einer Probe von Nucleinsäuren, die mittels eines Verfahrens nach Anspruch 1 oder 2 aus einer RNS-Startmatrix hergestellt wurden, wobei das Verfahren folgende Schritte aufweist:
(a) Herstellen einer Probe von Nucleinsäuren mittels eines Verfahrens nach einem der Ansprüche 1 bis 5;
(b) Inkontaktbringen der Probe mit einer Anordnung von Nucleinsäuren;
(c) Detektieren aller Bindungskomplexe an der Oberfläche der Anordnung, um Bindungskomplexdaten zu gewinnen; und
(d) Ermitteln der Anwesenheit des Nucleinsäureanalyten in der Probe unter Verwendung der Bindungskomplexdaten.

## Revendications

1. Procédé pour exécuter une réaction d'extension d'amorce à partir d'une matrice, ledit procédé comprenant :
la mise en contact d'une matrice d'ARN initiale avec :
(a) une composition d'amorce comprenant à la fois une amorce universelle qui inclut une pluralité de résidus dT séquentiels pour permettre l'hybridation avec la queue polyA d'ARN messagers, et au moins une amorce spécifique au gène de liaison ARN présentant une séquence qui la rend capable d'hybridation avec un ARN messager déterminé parmi les ARN messagers ou avec une partie de ceux-ci seulement, dans lequel l'amorce spécifique au gène inclut un ou plusieurs nucléotides mésappariés à son extrémité 3' ou dans lequel l'amorce spécifique au gène inclut un didésoxynucléotide 3', et
(b) une activité transcriptase inverse, dans des conditions de réaction d'extension d'amorce pour exécuter ladite réaction d'extension d'amorce à partir d'une matrice.

2. Procédé pour exécuter une réaction d'extension d'amorce à partir d'une matrice, ledit procédé comprenant :
(i) la mise en contact d'une matrice d'ARN initiale avec une composition d'amorce comprenant à la fois une amorce promoteur universelle qui inclut une région poly-dT pour l'hybridation avec la queue polyA d'ARN messagers et une région promoteur d'ARN polymérase 5' de la région poly-dT, et au moins une amorce spécifique au gène de liaison ARN présentant une séquence qui la rend capable d'hybridation avec un ARN messager déterminé parmi les ARN messagers ou avec une partie de ceux-ci seulement et qui est dépourvue d'une région promoteur d'ARN polymérase, pour produire des hybrides amorce-ARN messager ;
(ii) la conversion des hybrides amorce-ARN messager en produits ADN complémentaires double brin en mettant en contact les hybrides amorce-ARN messager avec une activité ARN polymérase ARN dépendante, une activité RNaseH et une activité ADN polymérase ADN dépendante, et
(iii) la transcription des produits ADN complémentaires double brin contenant une région promoteur d'ARN polymérase à double brin au moyen d'un ARN polymérase pour produire de l'ARN antisens.

3. Procédé suivant la revendication 2, dans lequel l'amorce spécifique au gène ne peut pas servir d'amorce pour l'extension d'amorce à partir d'une matrice.

4. Procédé suivant la revendication 1, 2 ou 3, dans lequel ladite amorce universelle et ladite amorce spécifique au gène à liaison ARN sont complémentaires de positions d'une seule espèce d'ARN qui sont séparées par moins de 15 nucléotides.

5. Procédé suivant l'une quelconque des revendications précédentes, dans lequel ladite composition d'amorce comprend une pluralité d'amorces spécifiques au gène.

6. Kit destiné à être utilisé pour mettre en oeuvre un procédé suivant la revendication 1, ledit kit comprenant :
(a) une amorce universelle qui inclut une pluralité de résidus dT séquentiels pour permettre l'hybridation avec la queue polyA d'ARN messagers, et
(b) au moins une amorce spécifique au gène présentant une séquence qui la rend capable d'hybridation avec un ARN messager déterminé parmi les ARN messagers ou avec une partie de ceux-ci seulement, dans lequel l'amorce spécifique au gène inclut un didésoxynucléotide 3'.

7. Kit destiné à être utilisé pour mettre en oeuvre un procédé suivant la revendication 2, ledit kit comprenant :
(a) une amorce promoteur universelle qui inclut (i) une région poly-dT pour l'hybridation avec la queue polyA d'ARN messagers, et (ii) une région promoteur d'ARN polymérase 5' de la région poly-dT, et
(b) au moins une amorce spécifique au gène présentant une séquence qui la rend capable d'hybridation avec un ARN messager déterminé parmi les ARN messagers ou avec une partie de ceux-ci seulement et qui est dépourvue d'une région promoteur d'ARN polymérase.

8. Kit suivant la revendication 6 ou 7, dans lequel ledit kit comprend en outre au moins une polymérase.

9. Procédé pour détecter la présence d'un analyte d'acide nucléique dans un échantillon d'acides nucléiques produit à partir d'une matrice d'ARN initiale au moyen d'un procédé suivant la revendication 1 ou 2, ledit procédé comprenant :
(a) la production dudit échantillon d'acides nucléiques au moyen d'un procédé suivant l'une quelconque des revendications 1 à 5 ;
(b) la mise en contact dudit échantillon avec un réseau d'acides nucléiques;
(c) la détection de tous complexes de liaison à la surface dudit réseau pour obtenir des données sur les complexes de liaison, et
(d) la détermination de la présence dudit analyte d'acide nucléique dans ledit échantillon en utilisant lesdites données sur les complexes de liaison.
